Europäisches Patentamt

European Patent Office

Office européen des brevets

(11) Veröffentlichungsnummer: **0 325 932**
**A2**

## (12) EUROPÄISCHE PATENTANMELDUNG

(21) Anmeldenummer: 89100335.2

(22) Anmeldetag: 10.01.89

(51) Int. Cl.4: **C07C 31/12 , C07C 31/125 , C07C 29/04**

(30) Priorität: 19.01.88 DE 3801275

(43) Veröffentlichungstag der Anmeldung:
02.08.89 Patentblatt 89/31

(84) Benannte Vertragsstaaten:
DE FR IT NL

(71) Anmelder: **EC ERDÖLCHEMIE GMBH**
**Postfach 75 20 02**
**D-5000 Köln 71(DE)**

(72) Erfinder: **Malessa, Reiner, Dr.**
**Naukler Strasse 57**
**D-7400 Tübingen(DE)**
Erfinder: **Schleppinghoff, Bernhard, Dr.**
**Adolf-Kolping-Strasse 5**
**D-4047 Dormagen 1(DE)**

(74) Vertreter: **Gerhards, Peter, Dr. et al**
**Bayer AG Konzernverwaltung RP**
**Patentabteilung**
**D-5090 Leverkusen 1 Bayerwerk(DE)**

(54) **Verfahren zur Herstellung von tertiären C4-C5-Alkoholen.**

(57) Tertiäre $C_4$-$C_5$-Alkohole können durch Hydratation von tertiären $C_4$-$C_5$-Olefinen an einem sauren Kationenaustauscher bei mäßig erhöhter Temperatur in der flüssigen Phase hergestellt werden; dem Einsatzgemisch werden 0,2-5 Gew.-%, bezogen auf das Gewicht des zu hydratisierenden tertiären Olefins, an tertiärem $C_4$-$C_5$-Alkohol zugesetzt.

EP 0 325 932 A2

EP 0 325 932 A2

## Verfahren zur Herstellung von tertiären C₄-C₅-Alkoholen

Die Erfindung betrifft ein Verfahren zur Herstellung von tertiären $C_4$-$C_5$-Alkoholen durch Hydratation von tertiären $C_4$-$C_5$-Olefinen an einem sauren Kationenaustauscher.

Aus US 2 813 908 ist es bereits bekannt, Olefine an Sulfonsäuregruppen enthaltenden Styrol-Divinylbenzol-Polymeren zu hydratisieren. Dieses Verfahren wird bei Temperaturen von 121 - 218°C und Drücken von bis zu 211 bar durchgeführt und führt bei Verwendung von i-Buten zu einer starken Bildung von i-Buten-Polymeren.

Aus US 3 257 469 ist weiterhin die Herstellung von tert.-Amylalkohol (TAA) an sauren, Sulfonsäuregruppen enthaltenden Ionenaustauschern durch Umsetzung von tert.-Amylen mit einem molaren Überschuß an Wasser bekannt. Hierzu wird beispielsweise bei einem Druck von 35 bar und Temperaturen von 66 - 107°C gearbeitet; eine weitergehende Hydratation (höherer Umsatz) wird durch die Mitverwendung von Lösungsmitteln erreicht, von denen i-Propanol oder Aceton besonders herausgestellt werden.

Dieses Verfahren umfaßt damit notwendigerweise die Abtrennung des mitverwendeten Lösungsmittels aus dem Reaktionsgemisch.

US 4 182 920 stellt eine Fortentwicklung des soeben beschriebenen Verfahrens dar und richtet sich ebenfalls bevorzugt auf die Herstellung von TAA. Als neu wird besonders herausgestellt, daß das gesamte Einsatzgemisch nur eine homogene Phase bildet und ein Teil des zu hydratisierenden Olefins in einen zweiten von mindestens insgesamt zwei Reaktoren eingespeist wird. Als Lösungsmittel wird speziell Aceton herausgestellt, das ausweislich der Ausführungsbeispiele in einer Menge von 60 -75 Gew.-% des gesamten Einsatzstroms vorliegt. Dieses Verfahren wird ebenfalls mit überschüssigen molaren Mengen an Hydratationswasser durchgeführt.

Die DE-OS 35 12 518 beschreibt die katalytische Hydratation niederer Olefine, wobei es sich ausweislich der Ausführungsbeispiele um Propen und n-Buten handelt. Die Reaktionsparameter sind 120 - 180°C und 40 - 200 bar Druck an einem stark sauren Kationenaustauscher in Gegenwart von überschüssigen molaren Wassermengen; das überschüssige Prozeßwasser wird recyclisiert. Unter diesen Bedingungen kommt es zu einem Ansteigen des Differenzdrucks im Reaktor, damit zu Druckstößen, die schließlich zu einem Abstellen der kontinuierlichen Reaktion zwingen. Bei der Hydratation von Propen wurden ferner Heißstellen im Katalysatorbett beobachtet, die zu übelriechenden Nebenprodukten führen. Zur Überwindung dieser Probleme beschreibt die genannte DE-OS den Zusatz eines stabilen Kationentensids, bei dem jedoch grundsätzlich die Möglichkeit der Desaktivierung des als Katalysator eingesetzten Kationenaustauschers beschrieben wird.

Es wurde nun ein Verfahren zur Herstellung von tertiären $C_4$-$C_5$-Alkoholen durch Hydratation von tertiären $C_4$-$C_5$-Olefinen an einem sauren Kationenaustauscher bei mäßig erhöhter Temperatur in der flüssigen Phase gefunden, das dadurch gekennzeichnet ist, daß man dem Einsatzgemisch 0,2 - 5 Gew.-%, bezogen auf das Gewicht des tertiären Olefins, an tertiärem $C_4$-$C_5$-Alkohol zusetzt.

Es wurde nämlich überraschenderweise gefunden, daß durch die erfindungsgemäße Maßnahme des Zusatzes von tertiärem $C_4$-$C_5$-Alkohol zum Einsatzgemisch wirksam die Bildung von Oligomeren unterdrückt werden kann. Das erfindungsgemäße Verfahren ist ferner ohne den Zusatz von Fremdstoffen durchführbar, die anschließend eine Abtrennung verlangen oder die unerwünschterweise im Produkt verbleiben. Der tertiäre $C_4$-$C_5$-Alkohol bildet sich im Verlaufe des Verfahrens aus dem zu hydratisierenden tertiären Olefin. Es ist daher umso überraschender, daß die grundsätzliche Gegenwart dieses tertiären Alkohols im Reaktionsgemisch nicht ausreicht, um die Bildung von Oligomeren und damit die Notwendigkeit ihrer Abtrennung zu verhindern, sondern daß es notwendig ist, den tertiären Alkohol in einer kleinen Menge bereits vor der Zuführung zum Hydratationskatalysator im Reaktionsgemisch zu haben.

Es ist grundsätzlich möglich, einem zu hydratisierenden tertiären $C_5$-Olefin t-Butylalkohol (TBA) zuzusetzen und umgekehrt dem zu hydratisierenden i-Buten TAA zuzusetzen. Im Sinne der Vermeidung von Fremdstoffen und deren Abtrennung ist es jedoch wirtschaftlich sinnvoll, den erfindungsgemäß dem Einsatzgemisch zuzusetzenden tertiären Alkohol mit der gleichen Anzahl von C-Atomen auszuwählen wie sie das zu hydratisierende Olefin hat.

Die Menge des zuzusetzenden tertiären $C_4$-$C_5$-Alkohols braucht nicht so groß zu sein, daß das Einsatzgemisch eine einzige homogene Phase bildet. Erfindungsgemäß werden dem Einsatzgemisch 0,2 - 5 Gew.-%, bevorzugt 0,3 -4 Gew.-%, besonders bevorzugt 1 - 3 Gew.-% des tertiären Alkohols zugesetzt, wobei alle Mengen auf das Gewicht des zu hydratisierenden tertiären Olefins bezogen sind.

Der zuzusetzende tertiäre Alkohol kann grundsätzlich in reiner Form zugesetzt werden. Da jedoch das Umsetzungsgemisch nach Verlassen des Reaktors diesen zuzusetzenden tertiären Alkohol als das gewünschte Reaktionsprodukt enthält, ist es eine vorteilhafte Variante des erfindungsgemäßen Verfahrens,

2

dem Einsatzgemisch eine solche Menge des im Verfahren anfallenden Reaktionsgemisches zuzusetzen, daß die gewünschte Menge an tertiärem Alkohol vor Beginn der katalytischen Hydratation vorliegt.

Die erfindungsgemäß einsetzbaren tertiären Olefine sind das i-Buten und die beiden i-Amylene (2-Methyl-buten-(1) und 2-Methyl-buten-(2)). Diese tertiären Olefine können für sich allein oder als Gemisch eingesetzt werden (wobei das Gemisch der tertiären Alkohole anfällt). Zur Vermeidung einer späteren Auftrennung werden die genannten Olefine jedoch bevorzugt getrennt nach C-Zahlen eingesetzt. Weiterhin können die genannten tertiären Olefine in reiner Form oder in Form von Destillationsschnitten, in denen sie vergesellschaftet mit anderen Kohlenwasserstoffen der gleichen C-Atomzahl vorkommen, eingesetzt werden. Aufgrund der dem Fachmann bekannten Gleichgewichtslage zur Bildung der tertiären Alkohole aus den zugehörigen tertiären Olefinen ist es jedoch bevorzugt, die tertiären Olefine in angereicherter oder in im wesentlichen reiner Form einzusetzen, da beim Einsatz eines Destillationsschnittes der gewünschte Alkohol in stark verdünnter Form enthalten würde. In weiterhin bevorzugter Form bezieht sich das erfindungsgemäße Verfahren auf den Einsatz der genannten i-Amylene zur Herstellung von TAA.

Ein weiterer wichtiger Aspekt des erfindungsgemäßen Verfahrens ist die Menge des einzusetzenden Hydratationswassers. Diese Wassermenge ist grundsätzlich unkritisch, so daß auch ein molarer Überschuß an Wasser tolerierbar ist. Die Untergrenze der Wassermenge wird im allgemeinen so angesetzt, daß im wesentlichen alles tertiäre Olefin zum zugehörigen tertiären Alkohol umgesetzt wird, das nach Lage des bereits erwähnten Umsetzungsgleichgewichtes zwischen dem Ausgangsolefin und dem gebildeten tertiären Alkohol umsetzbar ist. Wird diese Mindestmenge unterschritten, kommt es zu unnötigen Minderausbeuten, die umso schmerzlicher sind, als die dargestellte Gleichgewichtslage ohnehin auch vom theoretischen Standpunkt her nur eine begrenzte Ausbeute = Umsatz zum tertiären Alkohol erlaubt.

Es wurde weiterhin überraschenderweise festgestellt, daß eine wirksame Unterdrückung der Oligomerisierung des tertiären Olefins nur eintritt, wenn das Reaktionsgemisch Wassermengen enthält, die im wesentlichen ausreichen, um die von der Gleichgewichtslage her zulässige Hydratation auch zu ermöglichen. Weiterhin wurde gefunden, daß der Hydratationskatalysator eine höhere Aktivität zeigt, wenn die Wassermenge über die zur theoretischen Umsetzung nötige Menge im wesentlichen nicht hinausgeht. In bevorzugter Weise werden daher 80-120 %, besonders bevorzugt 90 -115 %, ganz besonders bevorzugt 95 - 105 % der Wassermenge zugesetzt, die zu einer nach der Lage des Gleichgewichtes theoretisch möglichen Hydratation führt. Die Lage des Gleichgewichtes ist von der gewählten Reaktionstemperatur abhängig, so daß die genannten prozentualen Wassermengen dieser Gleichgewichtslage angepaßt werden müssen. Die Veränderlichkeit des Gleichgewichtes mit der Reaktionstemperatur ist dem Fachmann bekannt, so daß er durch orientierende Versuche den erfindungsgemäß zu wählenden prozentualen Bereich der Wassermenge bestimmen kann. Wenn beispielsweise bei einer gewählten Reaktionstemperatur wegen der zugehörigen Gleichgewichtslage ein theoretischer Umsatz von 25 % des tertiären Olefins zu erwarten ist, beträgt die 80 -120 %ige Wassermenge, bezogen auf den zu erwartenden theoretischen Umsatz, 20 - 30 Mol-% Wasser, bezogen auf die gesamte molare Menge des tertiären Olefins im Einsatzgemisch. Zur Erhöhung der Reaktionsgeschwindigkeit kann es auch günstig sein, Verweilzeiten einzustellen, die nicht ganz zur Einstellung des Gleichgewichts ausreichen. Dann wird die benötigte Wassermenge auf den erzielbaren Umsatz an tertiärem Olefin berechnet.

Die bevorzugt zu hydratisierenden i-Amylene können beispielsweise als C$_5$-Destillationsschnitt (sogenannter Benzol-Vorlauf) oder in besonders bevorzugter Weise als im wesentlichen reines i-Amylen, beispielsweise aus der Spaltung von tert.-Amyl-methyl-ether, eingesetzt werden.

Als saure Kationenaustauscher können alle bekannten Typen, wie sulfonierte Phenol-Formaldehyd-Harze, sulfonierte Cumaron-Inden-Kondensationsprodukte, sulfonierte Polystyrole, sulfonierte Styrol-Divinylbenzol-Harze usw. eingesetzt werden; sie werden erfindungsgemäß in ihrer H$^+$-Form eingesetzt. In bevorzugter Weise werden erfindungsgemäß sulfonierte Styrol-Divinylbenzol-Harze mit einem Vernetzungsgrad (Gehalt an Divinylbenzol) von 2 -65 %, bevorzugt 8 - 25 %, eingesetzt. Solche sauren Kationenaustauscher sind dem Fachmann bekannt und unter vielen Bezeichnungen im Handel.

Die sauren Kationenaustauscher als Hydratationskatalysatoren werden mit einer Menge an gesamtem Einsatzgemisch beaufschlagt, die eine Raumgeschwindigkeit (LHSV = Liquid Hourly Space Velocity) von 0,05 - 1 Liter Reaktionsgemisch pro Liter Katalysator und Stunde, bevorzugt 0,1 - 0,4 L/L.h bedeutet. Das erfindungsgemäße Verfah ren wird bei einer Temperatur von 35 - 100°C, bevorzugt 50 - 80°C so durchgeführt, daß das Reaktionsgemisch im wesentlichen in der flüssigen Phase vorliegt. In jedem Fall arbeitet man bei einer Temperatur, die mehr als 5° unter dem Siedepunkt des tertiären Olefins beim eingestellten Reaktionsdruck liegt. Hierzu werden Drücke von 5 - 20 bar, bevorzugt 5 -15 bar angewandt. Der Druck an sich ist nicht kritisch für das erfindungsgemäße Verfahren und ist lediglich zur Aufrechterhaltung der flüssigen Phase erforderlich.

In Abhängigkeit von den verschiedenen Parametern und der davon abhängigen Gleichgewichtslage sind

3

TAA-Gehalte des Produkts im Bereich von 5 - 35 Massen-% erzielbar, typischerweise Gehalte im Bereich von 15 bis etwa 33 Massen-%, häufig 20-28 Massen-%.

Das erfindungsgemäße Verfahren kann in einfachen, dem Fachmann grundsätzlich bekannten Apparaten durchgeführt werden. So können beispielsweise die Einsatzstoffe tertiäres Olefin (gegebenenfalls ein dieses tertiäre Olefin enthaltendes Gemisch, etwa ein Destillationsschnitt), das Reaktionswasser und der erfindungsgemäß zuzusetzende tertiäre Alkohol getrennt oder nach Vorvermischung, jedoch gleichzeitig dem als Hydratationskatalysator dienenden sauren Kationenaustauscher zugeführt werden. Die erfindungsgemäße Umsetzung kann grundsätzlich auch absatzweise, etwa in einem Rührautoklaven, durchgeführt werden; die bevorzugte Verfahrensweise im technischen Maßstab ist jedoch die kontinuierliche. Nach Durchlaufen des Reaktors mit dem darin befindlichen Hydratationskatalysator wird das Reaktionsgemisch entspannt, wobei nicht umgesetztes tertiäres Olefin (gegebenenfalls im Gemisch mit anderen flüchtigen Bestandteilen) abgetrennt und bevorzugt recyclisiert wird. Das nach dieser Abtrennung verbleibende restliche Reaktionsgemisch wird sodann destillativ auf den reinen gewünschten tertiären Alkohol aufgearbeitet. In bevorzugter Weise wird dem Reaktionsgemisch vor der Aufarbeitung eine geringe Menge entzogen, die dem Einsatzgemisch als Quelle für den erfindungsgemäß miteinzusetzenden tertiären Alkohol dient. Bei der beschriebenen Verfahrensweise wird im allgemeinen unter einem solchen Druck gearbeitet, bei dem die Reaktionstemperatur unterhalb der Siedetemperatur des Reaktionsgemisches liegt.

Die geschilderte Reaktionsvariante kann dadurch verfeinert werden, daß der Hydratationskatalysator in mehreren hintereinanderliegenden Reaktoren angeordnet wird. Bei einer solchen mehrstufigen Durchführung, die in zwei, drei oder noch mehr Stufen, bevorzugt in zwei oder drei Stufen durchgeführt wird, ist es wiederum bevorzugt, von dem oben beschriebenen prozentualen Bereich an Reaktionswasser (80 - 120 %, bezogen auf den theoretisch zu erwartenden Gleichgewichtszustand) nur einen Teil in den ersten Reaktor einzuspeisen. Dieser Teil liegt bevorzugt bei 60 bis 90 Relativprozent des obigen prozentualen Bereiches. Das restliche Reaktionswasser wird dem zweiten Reaktor zugeführt, kann aber bei drei oder noch mehr Reaktionsstufen auch auf die Zuführung zum zweiten und zum dritten Reaktor (bzw. noch weiteren Reaktoren) aufgeteilt werden.

Die folgenden Ausführungsbeispiele verdeutlichen das erfindungsgemäße Verfahren beispielhaft, ohne es auf diese Beispiele einzuschränken.

Beispiel 1 (vgl. Tabelle)

In drei hintereinandergeschalteten, gleichgroßen Rohrreaktoren wurde reines i-Amylen mit Wasser unter Verwendung eines Sulfonsäuregruppen enthaltenden Styrol-Divinylbenzol-Harzes in der H$^+$-Form (Handelsprodukt SPC 118 der Bayer AG) unter 10 bar Überdruck umgesetzt. 70 % des insgesamt eingesetzten Wassers wurde vor, die restliche Menge nach dem 1. Reaktor eingespeist. Im Produkt wurden neben 21 % TAA 9,5 % unerwünschte Dimere gefunden.

Beispiel 2 (vgl. Tabelle)

Beispiel 1 wurde unter sonst gleichen Bedingungen wiederholt, jedoch mit der Ausnahme, daß dem als Rohstoff eingesetzten i-Amylen 1 % TAA zugesetzt wurde. Dieser Zusatz bewirkte eine Verminderung des Gehalts an Dimeren von 9,5 auf 3 %.

Beispiel 3 (vgl. Tabelle)

Beispiel 2 wurde mit einem TAA-Gehalt im eingesetzten i-Amylen von 2 % wiederholt. Die unerwünschte Dimerenbildung konnte dadurch auf 0,3 % gesenkt werden.

Tabelle

| Synthese bei verschiedenen Gehalten von TAA im Einsatzmaterial | | | |
|---|---|---|---|
| | Beispiel 1 | Beispiel 2 | Beispiel 3 |
| LHSV [$h^{-1}$] | 0,1 | 0,1 | 0,1 |
| Temperaturen [°C] | | | |
| Reaktor 1 | 65 | 65 | 65 |
| Reaktor 2 | 60 | 60 | 60 |
| Reaktor 3 | 60 | 60 | 60 |
| Druck [bar] | 10 | 10 | 10 |
| Einsatz | | | |
| Molverhältnis $H_2O$/i-Amylen | 1:4,8 | 1:4,8 | 1:4,8 |
| TAA im i-Amylen [Massen-%] | 0 | 1 | 2 |
| Produkt | | | |
| Dimere [Massen-%] | 9,5 | 3 | 0,3 |
| TAA [Massen-%] | 21 | 23 | 26 |

## Ansprüche

1. Verfahren zur Herstellung von tertiären $C_4$-$C_5$-Alkoholen durch Hydratation von tertiären $C_4$-$C_5$-Olefinen an einem sauren Kationenaustauscher bei mäßig erhöhter Temperatur in der flüssigen Phase, dadurch gekennzeichnet, daß man dem Einsatzgemisch 0,2 -5 Gew.-%, bezogen auf das Gewicht des tertiären Olefins, an tertiärem $C_4$-$C_5$-Alkohol zusetzt.

2. Verfahren nach Anspruch 1, dadurch gekennzeichnet, daß man 0,3 - 4 Gew.-% des tertiären Alkohols zusetzt.

3. Verfahren nach Anspruch 2, dadurch gekennzeichnet, daß man 1 - 3 Gew.-% des tertiären Alkohols zusetzt.

4. Verfahren nach Anspruch 1, dadurch gekennzeichnet, daß man den zuzusetzenden tertiären Alkohol in Form des beim Verfahren anfallenden Reaktionsgemisches, berechnet auf tertiären Alkohol, zusetzt.

5. Verfahren nach Anspruch 1, dadurch gekennzeichnet, daß das Hydratationswasser in einer molaren Menge von 80 - 120 %, bevorzugt 90 - 115 %, besonders bevorzugt 95 - 105 %, bezogen auf die zu erwartende Menge an tertiärem Alkohol, eingesetzt wird.

6. Verfahren nach Anspruch 1, dadurch gekennzeichnet daß bei einer Temperatur gearbeitet wird, die mehr als 5° unter der Siedetemperatur des tertiären Olefins beim eingestellten Reaktionsdruck liegt.

7. Verfahren nach Anspruch 1, dadurch gekennzeichnet, daß eine Raumgeschwindigkeit (LHSV = Liquid Hourly Space Velocity) von 0,05 - 1 Liter Einsatzgemisch pro Liter Katalysator und Stunde eingestellt wird.

8. Verfahren nach Anspruch 1, dadurch gekennzeichnet, daß aus tertiären $C_5$-Olefinen tert.-Amylalkohol (TAA) hergestellt wird und dem Einsatzgemisch 0,2 - 5 Gew.-%, bezogen auf das Gewicht des tertiären $C_5$-Olefins, an TAA zugesetzt werden.

9. Verfahren nach Anspruch 1, dadurch gekennzeichnet, daß bei mehrstufiger Verfahrensdurchführung 60 -90 % des gesamten Reaktionswassers vor der ersten Verfahrensstufe und der Rest nach der ersten Verfahrensstufe zugesetzt werden.